# FASCICULE DE BREVET EUROPEEN

(11) **EP 0 737 449 B1**
(45) Date de publication et mention de la délivrance du brevet: **15.12.1999**
(21) Numéro de dépôt: 96400588.8
(22) Date de dépôt: 21.03.1996
(51) Int. Cl.: A61B 17/70

(54) **Dispositif d'ostéosynthèse pour consolidation du rachis cervical**
Osteosynthesevorrichtung zum Zusammenheilen von Halswirbel
Osteosynthesis device for setting cervical vertebrae

(30) Priorité: 11.04.1995 FR 9504441
(43) Date de publication de la demande: 16.10.1996
(73) Titulaire: BIOMAT, 91892 Saclay (FR)
(72) Inventeur: Lahille, Michel, 91430 Vauhallan (FR); Lot, Guillaume, 75011 Paris (FR)
(74) Mandataire: Cabinet Martinet & Lapoux

(56) Documents cités:
- EP-A- 0 599 640
- EP-A- 0 608 623
- EP-A- 0 625 337
- DE-A- 3 916 198
- FR-A- 2 656 214
- FR-A- 2 687 561
- US-A- 4 773 402

## Description

La présente invention a trait à un dispositif d'ostéosynthèse pour consolider le rachis cervical.

Le rachis cervical est constitué de sept vertèbres. A partir du crâne, les deux premières vertèbres, l'atlas et l'axis, forment le rachis cervical supérieur. Les cinq dernières vertèbres forment le rachis cervical inférieur. Les deux premières vertèbres cervicales, atlas et axis, ont une fonction de stabilisation du crâne sur le rachis cervical et participent à la rotation du crâne sur son axe vertical.

Les cinq autres vertèbres cervicales constituant le rachis cervical inférieur participent à la flexion-extension et à l'inclinaison latérale du crâne. La stabilisation du rachis cervical par une instrumentation peut être effectuée, comme pour les autres segments rachidiens, par voie antérieure ou par voie postérieure.

Une intervention chirurgicale par voie antérieure est connue pour le rachis cervical inférieur avec différents types de plaques et de vis permettant des fixations étendues répondant aux besoins du patient.

En revanche, le rachis cervical supérieur nécessite une voie trans-orale pour être abordé par voie antérieure, c'est-à-dire un chemin à travers une cavité septique, ce qui provoque un risque septique majeur. Une intervention chirurgicale par voie antérieure est donc à proscrire pour le rachis cervical supérieur.

Des interventions par voie postérieure du rachis cervical supérieur sont connues. Différents dispositifs d'ostéosynthèse sont utilisés pour le rachis cervical inférieur seulement, ou s'étendent entre le rachis cervical inférieur et la partie postérieure du crâne naissant à l'occiput auquel l'atlas s'articule.

Un premier dispositif d'ostéosynthèse connu comprend des fils métalliques ou des crochets passant dans le canal rachidien, sous les lames des vertèbres constituant la partie postérieure du canal rachidien. Les fils ou les crochets sont solidarisés grâce à une tige assurant la stabilité de l'étendue du montage.

Ce premier dispositif d'ostéosynthèse nécessite l'intégrité des arcs postérieurs des vertèbres et n'est pas utilisable après une laminectomie, due à un traumatisme ou une tumeur. Par ailleurs, ce dispositif d'ostéosynthèse est généralement en acier inoxydable, interdisant tout examen ultérieur utilisant un scanner ou un système d'Imagerie par Résonance Magnétique IRM, examens pourtant indispensables pour le suivi de certains patients notamment en pathologie tumorale. En outre, l'implantation du premier dispositif d'ostéosynthèse est situé à proximité de la ligne médiane du rachis et donc tout au contact de la moelle épinière et rend souvent les contrôles délicats à interpréter, notamment ceux réalisés au moyen d'un système IRM.

Enfin sur un plan neurologique, l'implantation d'un crochet ou d'un fil de laçage à l'intérieur du canal rachidien réduit l'espace réservé à la moelle épinière et présente un risque neurologique.

D'autres dispositifs sont connus ne présentant pas ces inconvénients, mais sont conçus soit uniquement pour le rachis cervical supérieur, soit uniquement pour le rachis cervical inférieur ce qui limite leur utilisation à des pathologies bien précises.

La EP-A-0 608 623 (figures 35 à 40) divulgue un dispositif de fixation par tige du rachis cervical, comprenant deux assemblages symétriques constitués chacun d'un élément occipital et d'un élément cervical reliés par une tige.

L'élément occipital est composé de deux pièces distinctes, une plaque occipitale inférieure vissée sur l'occiput par deux vis et une plaque occipitale supérieure qui est superposée à la plaque inférieure et fixée à celle-ci par vissage d'un pion de centrage fileté. Le serrage des plaques par le pion permet également de serrer la tige entre deux rainures ménagées en vis-à-vis dans les plaques respectivement.

Cet assemblage pour former un élément occipital selon la EP-A-0 608 623 est composé de trois pièces distinctes et séparables. La plaque inférieure s'étend parallèlement à la tige et de part et d'autre du bras central où est ménagée la rainure pour recevoir la tige. Dans ces conditions, l'élément occipital selon la EP-A-0 608 623 présente une certaine fragilité au niveau de la liaison entre les trois pièces, et ne peut pas être positionné aisément sous l'occiput à cause de la forme doublement coudée de la tige, particulièrement en partie supérieure.

La présente invention vise à fournir un dispositif d'ostéosynthèse pour consolider le rachis cervical par voie postérieure qui remédie aux inconvénients précédents, c'est-à-dire qui soit plus résistant et positionnable aisément pour s'adapter d'une part aussi bien au rachis cervical inférieur qu'au rachis cervical supérieur, et d'autre part à un très grand nombre de pathologies, y compris après une laminectomie.

A cette fin, un dispositif d'ostéosynthèse pour consolidation du rachis cervical comprenant :
- une tige,
- un élément occipital relié à la tige, un premier moyen de liaison pour relier l'élément occipital à l'occiput et un second moyen de liaison pour relier l'élément occipital à la tige, et
- au moins un élément vertébral comprenant un troisième moyen de liaison pour relier l'élément vertébral à une vertèbre cervicale et un quatrième moyen de liaison pour relier l'élément vertébral à la tige,
est caractérisé en ce que l'élément occipital est composé de manière monolithique d'une plaque de contact pour être en contact avec l'occiput et d'un sabot situé à une extrémité longitudinale de la plaque de contact et traversé par la tige et fixé à celle-ci par le second moyen de liaison, la plaque de contact de l'élément occipital formant un angle d'environ 40° à 60° avec une partie de la tige qui traverse le sabot.

Grâce aux caractéristiques ci-dessus l'élément occipital est très résistant, tout en présentant une relative flexion, et peut-être positionné le long de la tige avec une lattitude très élevée.

Selon une première réalisation, la plaque de contact de l'élément occipital comprend une face plane destinée à être en contact avec l'occiput.

Selon une seconde réalisation, la plaque de contact de l'élément occipital comprend deux parties planes séparées par un coude ayant un angle obtu sensiblement inférieur de quelques degrés à un angle plat dans un plan axial longitudinal à la plaque de contact et à la tige. La forme de la plaque de contact est sélectionnée par le chirurgien pour une bonne adaptation à l'anatomie du cou du patient.

De préférence, le premier moyen de liaison comprend au moins un picot saillant de la plaque de contact.

En outre, le premier moyen de liaison comprend au moins une vis à visser dans l'occiput, traversant un trou ménagé dans la plaque de contact.

Selon une troisième réalisation, l'élément occipital comprend en outre un cinquième moyen de liaison pour relier l'élément occipital à une vertèbre cervicale. Le cinquième moyen de liaison comprend une griffe à insérer entre deux vertèbres cervicales.

Dans un autre aspect de l'invention, chacun des second et quatrième moyens de liaison comprend un alésage traversé par la tige et un moyen de pression pénétrant dans l'alésage pour immobiliser l'élément occipital, respectivement l'élément vertébral, sur la tige.

Avantageusement, la tige comprend un méplat contre lequel le moyen de pression s'appuie pour pousser la tige contre l'alésage.

De préférence, la tige est filetée et l'alésage est taraudé.

L'élément vertébral peut être un crochet vertébral, et le troisième moyen de liaison est alors une griffe à insérer entre deux vertèbres cervicales.

En variante, le troisième moyen de liaison comprend une plaque cervicale et au moins une vis à visser dans une vertèbre cervicale, traversant un trou ménagé dans la plaque cervicale.

L'élément vertébral peut encore être un élément de liaison transversale comprenant :
- une traverse ;
- deux troisièmes moyens de liaison pour lier l'élément de liaison transversale en deux régions d'une vertèbre cervicale, et
- deux quatrièmes moyens de liaison reliés par la traverse et solidarisés respectivement des troisièmes moyens de liaison pour relier l'élément de liaison transversale à deux tiges parallèles.

Selon une réalisation particulière, la traverse est sensiblement en arc de cercle, et les troisièmes moyens de liaison sont des griffes à insérer entre deux vertèbres cervicales, lesdites griffes formant entr'elles un angle d'environ 45° à 60°.

Le dispositif peut également comprendre en outre une plaque vertébrale longiligne indépendante de la tige et des vis traversant des trous ménagés dans la plaque vertébrale pour être vissées dans des vertèbres cervicales au-dessous de la tige.

D'autres caractéristiques et avantages de la présente invention apparaîtront plus clairement à la lecture de la description suivante de plusieurs réalisations préférées de l'invention en référence aux dessins annexés correspondants dans lesquels :
- la figure 1 est une vue latérale d'un dispositif d'ostéosynthèse situé dans un plan antéropostérieur du rachis cervical selon une première réalisation de l'invention;
- la figure 2 est une vue latérale d'un dispositif d'ostéosynthèse pour le rachis cervical selon une seconde réalisation de l'invention;
- la figure 3 est une vue arrière du dispositif de la figure 2;
- la figure 4 est une vue avant du dispositif de la figure 2;
- la figure 5 est une vue latérale d'un dispositif d'ostéosynthèse pour le rachis cervical selon une troisième réalisation de l'invention;
- la figure 6 est une vue en perspective d'un élément occipital du dispositif de la figure 6;
- la figure 7 est une vue en perspective du dispositif de la figure 1, complété par une plaque cervicale selon l'invention;
- la figure 8 est une vue en perspective d'un élément de liaison transversale selon l'invention; et
- la figure 9 est une vue postérieure de deux dispositifs d'ostéosynthèse analogues à celui de la figure 1 et reliés par l'élément de liaison transversale de la figure 8.

Les éléments du dispositif d'ostéosynthèse sont réalisés en titane pour permettre des examens ultérieurs au moyen d'un scanner ou d'un système IRM.

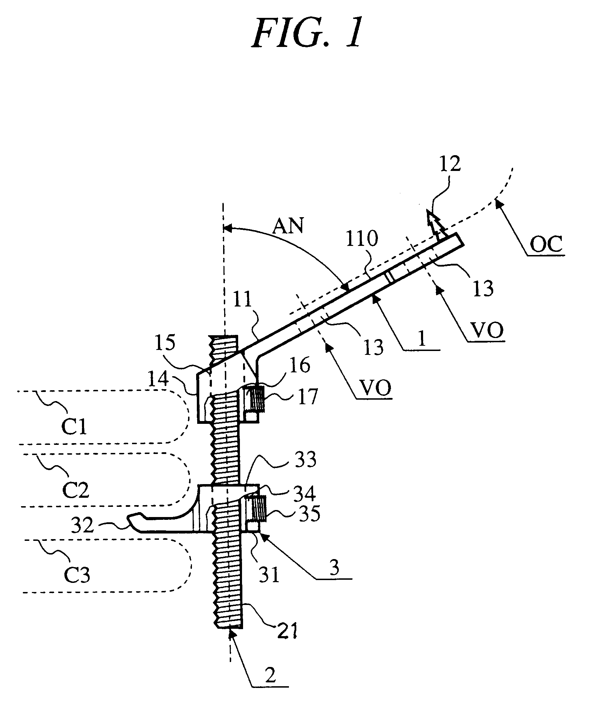

En référence à la figure 1, un dispositif d'ostéosynthèse selon une première réalisation de l'invention comprend un élément occipital 1, une tige de liaison 2 et un crochet vertébral 3.

L'élément occipital 1 comprend une plaque longiligne 11 présentant une face de contact plane 110 destinée à être appliquée contre l'occiput OC. Deux picots dentelés 12 saillent perpendiculairement sur une extrémité supérieure de la face de contact 110 pour pénétrer dans l'occiput.

Deux trous traversiers 13 à fraisure postérieure sont percés dans une portion centrale de la plaque d'élément occipitale 11. Une extrémité inférieure de la plaque 11 est constituée par un sabot sensiblement parallélépipédique 14 monolithique avec la plaque 11. Le sabot 14 est traversé par un alésage taraudé 15 dont l'axe longitudinal forme un angle AN d'environ 40 à 60° avec la plaque 11.

Un trou taraudé 16 est ménagé à partir de la face postérieure du sabot 14 sensiblement sous la plaque 11 et débouche radialement dans l'alésage 15. Une petite vis de pression 17 sans tête à six pans creux pénètre dans le trou taraudé 16.

La tige de liaison 2 est filetée et a un diamètre correspondant à l'alésage taraudé 15, c'est-à-dire égal ou sensiblement inférieur au diamètre de l'alésage 15. Un méplat 21 est formé sur toute la longueur de la tige 2 et constitue une surface d'appui notamment pour la vis de pression 17. En variante, la tige de liaison est lisse et coopère à glissement avec l'alésage 15 qui est alors également lisse.

Le crochet vertébral 3 comprend un sabot 31 et une griffe 32. Le sabot 31 est sensiblement parallélépipédique. Un alésage taraudé 33 propre à être traversé par la tige de liaison 2 est ménagé dans le sabot 31. Un trou taraudé 34 analogue au trou 16 de la plaque occipitale 1 débouche radialement dans l'alésage 33. Une vis de pression 35 analogue à la vis 17 est vissée dans le trou 34. En variante, l'alésage 33 est lisse et le crochet vertébral 3 coulisse sur la tige 2 jusqu'à une position souhaitée à laquelle la vis 35 est vissée pour bloquer le crochet 3 sur la tige 2.

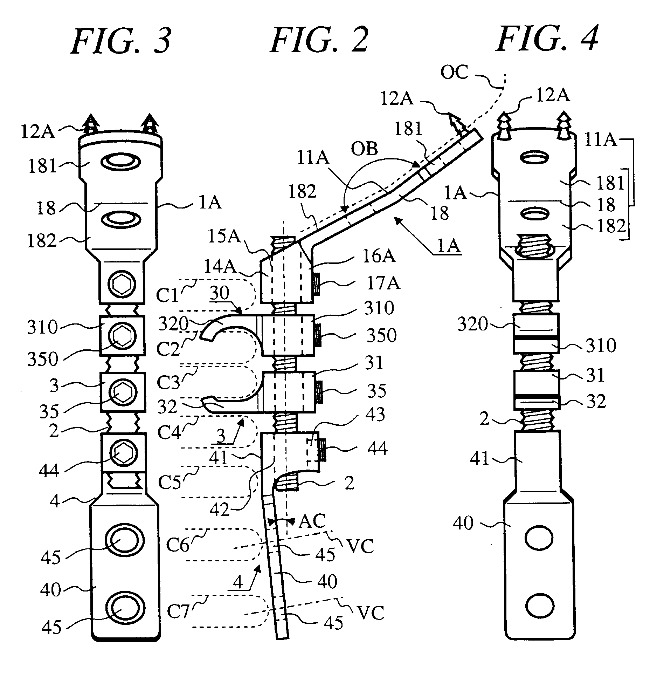

La griffe 32 du crochet vertébral 3 s'étend sensiblement perpendiculairement à l'axe de l'alésage 33. Selon la réalisation illustrée à la figure 1, la griffe 32 a une première extrémité accolée au sabot, qui est sensiblement en forme de dièdre à sommet arrondi, une partie centrale rectiligne, et une seconde extrémité libre, légèrement recourbée vers le haut. Selon une autre variante, comme montré en 320 dans la figure 2, la griffe 32 a un profil sensiblement en demi-croissant.

Lorsque le chirurgien a implanté le dispositif de la figure 1 dans le cou d'un patient, la face de contact 110 de la plaque occipitale 11 est en contact avec l'occiput OC du patient. Les picots 12 sont plantés dans l'occiput. Deux vis trans-osseuses autotaraudeuses à tête fraisée schématisées par des axes VO dans la figure 1 sont en outre vissées dans l'écaille occipitale, à travers les trous 13. Les picots 12 et les vis VO contribuent à une fixation ferme de la plaque occipitale contre l'occiput du patient.

La tige de liaison 2 est vissée ou engrenée dans le taraudage de l'alésage 15 du sabot 14 de l'élément occipital 1. Puis après positionnement convenable de l'extrémité supérieure de la tige 2 par rapport à l'occiput, la vis 17 est vissée dans le trou taraudé 16 et pressée contre le méplat 21 de la tige 2 de manière à pousser la tige 2 contre l'alésage 15, et particulièrement les filets de la tige 2 contre ceux de l'alésage taraudé 15, pour bloquer en translation et en rotation la tige 2 par rapport à l'élément occipital 1, en addition de la coopération entre le filetage de la tige 2 et du trou taraudé 15.

La tige de liaison 2 est également insérée dans l'alésage 33 du crochet vertébral 3 et est bloquée en translation et en rotation par la vis de pression 35 vissée dans le trou taraudé 34 jusqu'à venir en appui contre le méplat de tige 21. La griffe 32 du crochet vertébral 3 est insérée entre les seconde et troisième vertèbres cervicales C2 et C3 et accrochée à un évidement ou cavité vertébral. Après vissage des vis 17 et 35, l'élément occipital 1, la tige de liaison 2 et le crochet vertébral 3 forment un ensemble rigide qui stabilise le rachis cervical supérieur. La distance relative entre l'élément occipital 1 et le crochet vertébral 3 le long de la tige de liaison 2 ainsi que l'orientation de l'élément occipital 1 et du crochet vertébral 3 autour de l'axe de la tige de liaison 2 sont réglées en condition péropératoire par le chirurgien.

En référence aux figures 2 à 4, une seconde réalisation du dispositif d'ostéosynthèse selon l'invention comprend un élément occipital 1A, la tige de liaison 2, le crochet vertébral 3, un second crochet vertébral 30, et un élément cervical 4.

L'élément occipital 1A est longiligne et présente une plaque de contact 11A ayant un coude faiblement prononcé la partageant en deux parties planes 181 et 182 formant entr'elles un angle obtu OB orienté vers le haut, sensiblement inférieur de quelques degrés à un angle plat de 180°. La première partie plane 181 s'étend entre une extrémité supérieure de l'élément occipital 1A munie de picots 12A saillant perpendiculairement sur la face de contact 110A avec l'occiput, et le coude 18 situé sensiblement au milieu de la plaque 11A. La seconde partie 182 s'étend entre sensiblement le coude 18 de la plaque 11A et une seconde extrémité de la plaque 11A reliée à un sabot 14A.

Le sabot 14A est analogue au sabot 14 de la plaque occipitale 1 et est monolithique avec la plaque 1A. Un alésage taraudé 15A est formé dans le sabot 14A. L'axe longitudinal de l'alésage forme un angle AN d'environ 40 à 60° avec la seconde partie 182 de la plaque 11A. Un trou taraudé 16A est ménagé dans le sabot 14A pour déboucher radialement dans l'alésage taraudé 15A. Le trou taraudé est ménagé à partir d'une face postérieure du sabot située sous la plaque occipitale 1A. Une vis de pression 17A est vissée dans le trou taraudé 16A.

En variante, la plaque de contact présente une face de contact légèrement courbée entre le sabot et l'extrémité opposée munie de picots, selon un rayon de courbure propre à épouser sensiblement le profil postérieur de l'occiput du patient.

Le crochet vertébral 30 est analogue au crochet vertébral 3 et comprend un sabot 310 et une griffe 320. Le sabot 310 comprend un alésage taraudé pour recevoir la tige 2 et un trou taraudé pour recevoir une vis de pression 350. L'extrémité antérieure de la griffe est courbée vers le bas, c'est-à-dire dans une direction opposée à celle de la griffe du crochet 3 qui est situé en dessous du crochet 30, lequel est situé en dessous du sabot 14A de l'élément occipital.

La griffe du crochet 30 est à insérer au-dessus d'une vertèbre cervicale, par exemple la seconde vertèbre cervicale C2. La griffe du crochet 3 est à insérer au-dessous de la seconde vertèbre cervicale C2, ou de la troisième vertèbre cervicale C3 comme illustré à la figure 2. Après blocage des crochets 3 et 30 par vissage des vis de pression 35 et 350 les crochets 3 et 30 sont ancrés en encadrant la vertèbre C2, ou les vertèbres C2 et C3.

L'élément cervical 4 comprend une plaque longiligne et sensiblement rectiligne 40 qui s'étend vers le bas à partir d'un sabot 41. Le sabot 41 comprend un moyen de liaison à la tige de liaison 2 analogues à ceux des sabots des crochets vertébraux 3 et 30 et de l'élément vertébral 1A. Le moyen de liaison comprend un alésage taraudé 42 traversé par la tige de liaison 2, un trou taraudé 43 débouchant radialement dans l'alésage 42, et une vis de pression 44 vissée dans le trou taraudé 43 et pénétrant dans l'alésage taraudé 42.

La plaque cervicale 40 s'étend dans une direction formant un angle AC de quelques degrés avec l'axe de la tige de liaison 2, lorsque l'élément cervical 4 et la tige de liaison 2 sont assemblées comme sur les figures 2 à 4.

Deux trous traversiers 45 à fraisure postérieure sont ménagés dans la plaque vertébrale 4. Les trous 45 sont centrés sur un axe longitudinal de la plaque 4 et sont espacés entr'eux d'une distance correspondant sensiblement à celle entre des corps vertébraux de deux vertèbres cervicales C5 et C6, C6 et C7.

La plaque cervicale 40 est solidarisée avec des vertèbres par des vis schématisés par des axes VC, insérées dans les trous 45 et vissées dans les corps vertébraux des vertèbres.

En variante, la plaque cervicale 40 est sensiblement deux fois plus courte à partir du sabot 41 et comprend un seul trou 45. Selon une autre variante, la plaque cervicale 4 est sensiblement une fois et demi plus longue et comprend trois trous 45 régulièrement espacés.

Lors de l'assemblage du dispositif d'ostéosynthèse selon les figures 2 à 4, le chirurgien règle les positions relatives des différents éléments 1A, 30, 3 et 4, notamment en hauteur le long et en orientation autour de l'axe de la tige de liaison 2. En variante, le crochet supérieur 30 est supprimé.

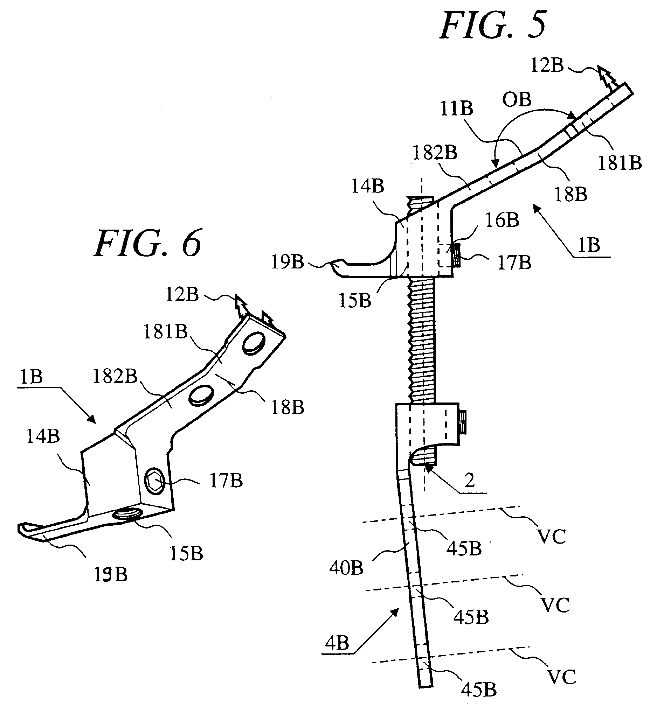

En référence aux figures 5 et 6, un dispositif d'ostéosynthèse selon une troisième réalisation de l'invention comprend un élément occipital 1B, la tige de liaison 2 et un élément cervical 4B.

L'élément occipital 1B est analogue à une combinaison monolithique de l'élément occipital lA et du crochet vertébral 3. Plus précisément, l'élément occipital 1B comprend une plaque occipitale 11B pour être en contact avec l'occiput. Deux parties planes 181A et 182B de la plaque 11B forment entr'elles un coude obtu 18B. A une extrémité supérieure, la plaque occipitale 11B comprend des picots 12B saillant perpendiculairement sur la face de la plaque 11B en contact avec l'occiput. A une extrémité inférieure, l'élément occipital 1B comprend un sabot 14B. Le sabot 14B comprend des moyens de liaison avec la tige de liaison 2, sous la forme d'un alésage taraudé 15B traversé par la tige de liaison 2, et un trou 16B radial à l'alésage 15B, et une vis de pression 17B pénétrant dans le trou 16B pour bloquer en translation et rotation la tige de liaison 2 dans l'alésage 15B.

Une griffe 19B saille à partir du sabot 14B, dans une direction sensiblement opposée à celle de la plaque occipitale 11B. La griffe 19B est analogue à la griffe 32 du crochet vertébral 3.

L'élément cervical 4B est analogue à l'élément cervical 4 précédemment décrit en référence aux figures 2 à 4, mais présente une plaque 40B plus longue que la plaque 40. Trois trous 45B sont ménagés dans la plaque 40B pour recevoir des vis VC à visser dans des corps vertébraux de vertèbres cervicales C3 à C7.

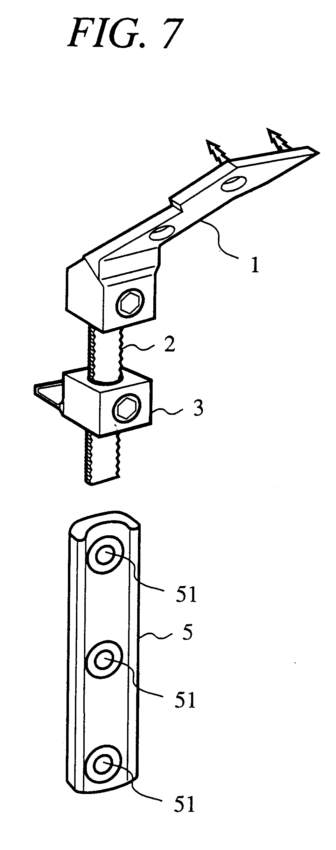

En référence à la figure 7, le dispositif d'ostéosynthèse selon la première réalisation est complété par une plaque cervicale 5 indépendante de la tige de liaison 2 et par conséquent du crochet vertébral 3 et de l'élément occipital 1.

La plaque 5 comprend trois trous 51 à fraisure postérieure régulièrement espacés pour recevoir des vis à tête fraisée à visser dans des corps vertébraux. Après implantation, la plaque 5 est au-dessous du dispositif d'ostéosynthèse selon la première réalisation. En variante, la plaque 5 comprend deux ou quatre trous 51.

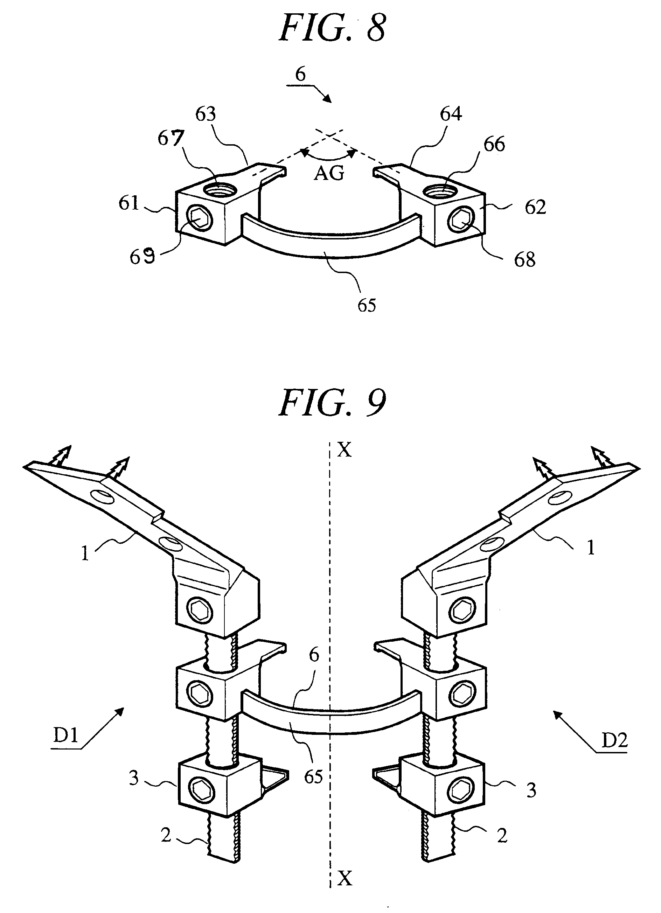

En référence à la figure 8, un élément de liaison transversale 6 comprend deux sabots identiques 61 et 62 à partir desquels saillent deux griffes 63 et 64, et une traverse 65 sensiblement en arc de cercle reliant les deux sabots 61 et 62.

Les sabots 61 et 62 sont analogues au sabot 31 du crochet 3. Le sabot 61, 62 comprend un alésage taraudé 67, 66 pour être traversé par une tige de liaison et un trou taraudé pour recevoir une vis de pression 69, 68. Les griffes 63 et 64 sont analogues à la griffe 32 du crochet 3 ou à la griffe 320 du crochet 30.

La traverse 65 relie des faces latérales respectives des sabots 61 et 62 de sorte que les griffes 63 et 64 sont dirigées selon des axes sécants formant entr'eux un angle AG de 45 à 60° environ et peuvent être liés à deux régions distinctes d'une vertèbre cervicale.

L'élément de liaison transversale 6 relie deux dispositifs d'ostéosynthèse D1 et D2 selon l'invention. Ces deux dispositifs peuvent être identiques et composés respectivement d'un élément occipital 1, d'une tige de liaison 2 et d'un crochet vertébral 3 selon la réalisation illustrée à la figure 9. Vu de l'arrière, l'un D1 des dispositifs est implanté sensiblement à gauche d'un axe médian X-X du rachis cervical et l'autre dispositif D2 est implanté sensiblement à droite de l'axe X-X.

L'élément de liaison transversale 6 est vissé et bloqué sur les deux tiges de liaison 2 des dispositifs D1 et D2.

Le chirurgien choisit les dispositifs gauche et droit à relier, et ceux-ci ne sont pas nécessairement identiques mais sont adaptés aux besoins du patient.

Le chirurgien choisit un élément occipital 1, 1A ou 1B, et au moins un élément tel qu'un crochet 3 ou 30, ou bien un élément cervical 4 ou 4B, et les assemble par une tige de liaison 2, dont la longueur est adaptable à la région traumatique ou tumorale du patient. A la place d'un élément cervical 4 ou 4B, le chirurgien peut choisir une plaque cervicale 5.

Le chirurgien implante généralement deux dispositifs gauche et droit qui sont reliés par un élément de liaison transversale 6.

De nombreuses combinaisons des éléments décrits sont réalisables, de sorte que le dispositif s'adapte à un grand nombre de pathologies.

## Revendications

1. Dispositif d'ostéosynthèse pour consolidation du rachis cervical, comprenant :
- une tige (2),
- un élément occipital (1, 1A, 1B) relié à la tige, un premier moyen de liaison (12, 12A, 12B) pour relier l'élément occipital à l'occiput (OC) et un second moyen de liaison (15, 16, 17) pour relier l'élément occipital à la tige (2), et
- au moins un élément vertébral (3, 30, 4, 4B, 6) comprenant un troisième moyen de liaison (32, 320, 45, 45B, VC, 63, 64) pour relier l'élément vertébral à une vertèbre cervicale (C1 - C7) et un quatrième moyen de liaison (33, 34, 35, 42, 43, 44, 65, 66, 67, 68) pour relier l'élément vertébral à la tige (2),
caractérisé en ce que l'élément occipital (1, 1A, 1B) est composé de manière monolithique d'une plaque de contact (11, 11A, 11B) pour être en contact avec l'occiput (OC) et d'un sabot (14, 14A, 14B) situé à une extrémité longitudinale de la plaque de contact et traversé par la tige (2) et fixé à celle-ci par le second moyen de liaison, la plaque de contact (11, 11A, 11B) de l'élément occipital (1, 1A, 1B) formant un angle (AN) d'environ 40° à 60° avec une partie de la tige (2) qui traverse le sabot.

2. Dispositif d'ostéosynthèse conforme à la revendication 1, caractérisé en ce que la plaque de contact (11A, 11B) de l'élément occipital (1A, 1B) comprend deux parties planes (181, 182, 181B, 182B) séparées par un coude (18, 18B) ayant un angle obtu (OB) sensiblement inférieur de quelques degrés à un angle plat dans un plan axial longitudinal à la plaque de contact et à la tige.

3. Dispositif d'ostéosynthèse conforme à la revendication 1 ou 2, caractérisé en ce que le premier moyen de liaison comprend au moins0 un picot (12, 12A, 12B) saillant de la plaque de contact (11, 11A, 11B).

4. Dispositif d'ostéosynthèse conforme à l'une quelconque des revendications 1 à 3, caractérisé en ce que le premier moyen de liaison comprend au moins une vis (VO) à visser dans l'occiput (OC), traversant un trou (13) ménagé dans la plaque de contact (11, 11A, 11B).

5. Dispositif d'ostéosynthèse conforme à l'une quelconque des revendications 1 à 4, caractérisé en ce que l'élément occipital (1B) comprend en outre un cinquième moyen de liaison (19B) pour relier l'élément occipital à une vertèbre cervicale (C1, C2).

6. Dispositif d'ostéosynthèse conforme à la revendication 5, caractérisé en ce que le cinquième moyen de liaison comprend une griffe (19B) à insérer entre deux vertèbres cervicales.

7. Dispositif d'ostéosynthèse conforme à l'une quelconque des revendications 1 à 6, caractérisé en ce que chacun des second et quatrième moyens de liaison comprend un alésage (15, 33) traversé par la tige (2) et un moyen de pression (17, 35) pénètrant dans l'alésage pour immobiliser l'élément occipital, (1), respectivement l'élément vertébral (3), sur la tige.

8. Dispositif d'ostéosynthèse conforme à revendication 7, caractérisé en ce que la tige (2) comprend un méplat (21) contre lequel le moyen de pression (17, 35) s'appuie pour pousser la tige (2) contre l'alésage (15, 33).

9. Dispositif conforme à la revendication 7 ou 8, caractérisé en ce que la tige (2) est filetée et l'alésage (15, 33) est taraudé.

10. Dispositif conforme à l'une quelconque des revendications 1 à 9, caractérisé en ce que l'élément vertébral est un crochet vertébral (3, 30), et le troisième moyen de liaison est une griffe (32, 320) à insérer entre deux vertèbres cervicales (C1 - C7).

11. Dispositif conforme à l'une quelconque des revendications 1 à 9, caractérisé en ce que l'élément vertébral comprend une plaque cervicale (40, 40B) et au moins une vis à visser dans une vertèbre cervicale (C1 - C7), traversant un trou (45, 450) ménagé dans la plaque cervicale.

12. Dispositif conforme à l'une quelconque des revendications 1 à 9, caractérisé en ce que l'élément vertébral est un élément de liaison transversale (6) comprenant :
- une traverse (65) ;
- deux troisièmes moyens de liaison (63, 64) pour lier l'élément de liaison transversale en deux régions d'une vertèbre cervicale, et
- deux quatrièmes moyens de liaison reliés par la traverse (65) et solidarisés respectivement des troisièmes moyens de liaison pour relier l'élément de liaison transversale à deux tiges parallèles (2).

13. Dispositif conforme à la revendication 12, caractérisé en ce que la traverse (65) est sensiblement en arc de cercle, et les troisièmes moyens de liaison sont des griffes (63, 64) à insérer entre deux vertèbres cervicales (C1 - C7), lesdites griffes formant entr'elles un angle (AG) d'environ 45° à 60°.

## Claims

1. An osteosynthesis device for consolidating the cervical column, comprising:
- a rod (2),
- an occipital element (1, 1A, 1B) connected to the rod, a first connecting means (12, 12A, 12B) for connecting the occipital element to the occiput (OC) and a second connecting means (15, 16, 17) for connecting the occipital element to the rod (2), and
- at least one vertebral element (3, 30, 4, 4B, 6) comprising a third connecting means (32, 320, 45, 45B, VC, 63, 64) for connecting the vertebral element to a cervical vertebra (C1 - C7) and a fourth connecting means (33, 34, 35, 42, 43, 44, 65, 66, 67, 68) for connecting the vertebral element to the rod (2),
characterised in that the occipital element (1, 1A, 1B) has a monolithic construction in the form of a contact plate (11, 11A, 11B) so as to be in contact with the occiput (OC) and a shoe (14, 14A, 14B) situated at one longitudinal end of the contact plate and through which the rod (2) extends and fastened to the rod by the second connecting means, the contact plate (11, 11A, 11B) of the occipital element (1, 1A, 1B) being at an angle (AN) of about 40° to 60° to a part of the rod (2) which extends through the shoe.

2. An osteosynthesis device according to claim 1, characterised in that the contact plate (11A, 11B) of the occipital element (1A, 1B) comprises two flat parts (181, 182, 181B, 182B) separated by a bend (18, 18B) having an obtuse angle (OB) substantially less, by a few degrees, than a straight angle in a longitudinal axial plane to the contact plate and the rod.

3. An osteosynthesis device according to claim 1 or 2, characterised in that the first connecting means comprises at least one barb (12, 12A, 12B) projecting from the contact plate (11, 11A, 11B).

4. An osteosynthesis device according to any of claims 1 to 3, characterised in that the first connecting means comprises at least one screw (VO) for screwing into the occiput (OC) through a hole (13) formed in the contact plate (11, 11A, 11B).

5. An osteosynthesis device according to any of claims 1 to 4, characterised in that the occipital element (1B) also comprises a fifth connecting means (19B) for connecting the occipital element to a cervical vertebra (C1, C2).

6. An osteosynthesis device according to claim 5, characterised in that the fifth connecting means comprises a claw (19B) for inserting between two cervical vertebrae.

7. An osteosynthesis device according to any of claims 1 to 6, characterised in that each of the second and fourth connecting means has a bore (15, 33) through which the rod (2) extends and a pressure means (17, 35) which extends into the bore in order to immobilise the occipital element (1) and the vertebral element (3) respectively on the rod.

8. An osteosynthesis device according to claim 7, characterised in that the rod (2) has a flat (21) against which the pressure means (17, 35) bears in order to push the rod (2) against the bore (15, 33).

9. A device according to claim 7 or 8, characterised in that the rod (2) is screwthreaded and the bore (15, 33) is tapped.

10. A device according to any of claims 1 to 9, characterised in that the vertebral element is a vertebral hook (3, 30) and the third connecting means is a claw (32, 320) for inserting between two cervical vertebrae (C1 - C7).

11. A device according to any of claims 1 to 9, characterised in that the vertebral element comprises a cervical plate (40, 40B) and at least one screw for screwing into a cervical vertebra (C1 - C7) through a hole (45, 450) formed in the cervical plate.

12. A device according to any of claims 1 to 9, characterised in that the vertebral element is a transverse connecting element (6) comprising:
- a cross-member (65);
- two second connecting means (63, 64) for connecting the transverse connecting element to two regions of a cervical vertebra, and
- two fourth connecting means connected by the cross-member (65) and secured to respective third connecting means in order to Connect the transverse connecting element to two parallel rods (2).

13. A device according to claim 12, Characterised in that the cross-member (65) is substantially in the form of an arc of a circle and the third connecting means are claws (63, 64) for inserting between two cervical vertebrae (C1 - C7), the said claws forming an angle (AG) of approximately 45° to 60° between them.

## Patentansprüche

1. Osteosynthesevorrichtung zur Konsolidierung der Halswirbelsäule, umfassend
- eine Spindel (2),
- ein an der Spindel befestigtes Okzipitalteil (1, 1A, 1B), ein erstes Befestigungsmittel (12, 12A, 12B) zur Befestigung des Okzipitalteils am Hinterhaupt (OC) und ein zweites Befestigungsmittel (15, 16, 17) zur Befestigung des Okzipitalteils an der Spindel (2), und
- mindestens ein Wirbelteil (3, 30, 4, 4B, 6), ein drittes Befestigungsmittel (32, 320, 45, 45B, VC, 63, 64) zur Befestigung des Wirbelteils an einem Halswirbel (C1 - C7) und ein viertes Befestigungsmittel (33, 34, 35, 42, 43, 44, 65, 66, 67, 68) zur Befestigung des Wirbelteils an der Spindel (2) umfassend,
dadurch gekennzeichnet, dass das Okzipitalteil (1, 1A, 1B) einteilig aus einer Kontaktplatte (11, 11A, 11B) zur Berührung mit dem Hinterhaupt (OC) und einem Schuh*** (14, 14A, 14B) besteht, der sich an einem Ende in der Längsrichtung der Kontaktplatte befindet, durch den die Spindel (2) verläuft und der durch das zweite Befestigungsmittel an dieser befestigt ist, wobei die Kontaktplatte (11, 11A, 11B) des Okzipitalteils (1, 1A, 1B) einen Winkel (AN) von ungefähr 40° bis 60° mit einem Teil der Spindel (2) bildet, die den Schuh durchquert.

2. Osteosynthesevorrichtung nach Patentanspruch 1, dadurch gekennzeichnet, dass die Kontaktplatte (11A, 11B) des Okzipitalteils (1A, 1B) zwei ebene Teile (181, 182, 181B, 182B), getrennt durch einen Knick (18, 18B) mit einem stumpfen Winkel (OB), der im wesentlichen um einige Grad kleiner ist, als ein ebener Winkel in einer Axialebene längs der Kontaktplatte und der Spindel, umfasst.

3. Osteosynthesevorrichtung nach Patentanspruch 1 oder 2, dadurch gekennzeichnet, dass das erste Befestigungsmittel mindestens einen Stachel (12, 12A, 12B) umfasst, der aus der Kontaktplatte (11, 11A, 11B) herausragt.

4. Osteosynthesevorrichtung nach irgendeinem der Patentansprüche 1 bis 3, dadurch gekennzeichnet, dass das erste Befestigungsmittel mindestens eine Schraube (VO) zum Einschrauben ins Hinterhaupt (OC) umfasst, die ein Loch (13) durchquert, das in der Kontaktplatte (11, 11A, 11B) ausgebildet ist.

5. Osteosynthesevorrichtung nach irgendeinem der Patentansprüche 1 bis 4, dadurch gekennzeichnet, dass das Okzipitalteil (10) außerdem ein fünftes Befestigungsmittel (19B) umfasst, um das Okzipitalteil mit einem Halswirbel (C1, C2) zu verbinden.

6. Osteosynthesevorrichtung nach Patentanspruch 5, dadurch gekennzeichnet, dass das fünfte Befestigungsmittel einen Greifer (19B) zum Einführen zwischen zwei Halswirbel umfasst.

7. Osteosynthesevorrichtung nach irgendeinem der Patentansprüche 1 bis 6, dadurch gekennzeichnet, dass das zweite und das Vierte Befestigungsmittel eine Bohrung (15, 33) umfasst, die von der Spindel (2) durchquert wird, und ein Druckmittel (17, 35), das in die Bohrung eindringt, um das Okzipitalteil (1) bzw. das Wirbelteil (3) an der Spindel fest zu setzen.

8. Osteosynthesevorrichtung nach Patentanspruch 7, dadurch gekennzeichnet, dass die Spindel (2) eine Anflachung (21) umfasst, gegen die das Druckmittel (17, 35) drückt, um die Spindel (2) gegen die Bohrung (15, 33) zu drücken.

9. Vorrichtung nach Patentanspruch 7 oder 8, dadurch gekennzeichnet, dass die Spindel (2) gewindet ist und die Bohrung (15, 33) innengewindet.

10. Vorrichtung nach irgendeinem der Patentansprüche 1 bis 9, dadurch gekennzeichnet, dass das Wirbelteil ein Wirbelhaken (3, 30) ist und das dritte Befestigungsmittel ein Greifer (32, 320) zum Einführen zwischen zwei Halswirbel (C1 - C7).

11. Vorrichtung nach irgendeinem der Patentansprüche 1 bis 9, dadurch gekennzeichnet, dass das Wirbelteil eine Zervikalplatte (40, 40B) und mindestens eine Schraube zum Einschrauben in einen Halswirbel (C1 - C7) umfasst, die ein Loch (45, 450) durchquert, das in der Zervikalplatte ausgebildet ist.

12. Vorrichtung nach irgendeinem der Patentansprüche 1 bis 9, dadurch gekennzeichnet, dass das Wirbelteil ein Querverbindungsteil (6) ist, umfassend:
- eine Querstrebe (65),
- zwei dritte Befestigungsteile (63, 64), um das Querbefestigungsteil in zwei Bereichen eines Halswirbels zu befestigen, und
- zwei vierte Befestigungsteile, die durch die Querstrebe (65) verbunden sind und jeweils an den dritten Befestigungsmitteln befestigt sind, um das Querbefestigungsteil mit zwei parallelen Spindeln (2) zu verbinden.

13. Vorrichtung nach Patentanspruch 12, dadurch gekennzeichnet, dass die Querstrebe (65) im wesentlichen ein Kreisbogen ist und die dritten Befestigungsmittel Greifer (63, 64) zum Einführen zwischen zwei Halswirbel (C1 - C7), wobei die genannten Greifer einen Winkel (AG) von ungefähr 45° bis 60° einschließen.
